# FASCICULE DE BREVET EUROPEEN

(11) **EP 2 273 895 B1**
(45) Date de publication et mention de la délivrance du brevet: **14.12.2016**
(21) Numéro de dépôt: 09729216.3
(22) Date de dépôt: 24.03.2009
(51) Int. Cl.: A23L 33/00, A23L 33/10

(54) **COMPOSITIONS COMPRENANT DE L'ACIDE MYRISTIQUE, DES ACIDES GRAS COMPRENANT UN DIENE CONJUGUE N-5CIS, N-7TRANS OU UN TRIENE CONJUGUE N-5CIS, N-7TRANS, N-9CIS**
ZUSAMMENSETZUNG ENTHALTEND MYRISTINSÄURE, FETTSÄUREN BESTEHEND AUS EINEM N-5CIS, N7-TRANS KONJUGIERTEN DIEN ODER EINEM N-5CIS, N-7TRANS, N9CIS KONJUGIERTEN TRIEN
COMPOSITIONS COMPRISING MYRISTIC ACID, FATTY ACIDS COMPRISING A CONJUGATED DIENE N 5CIS, N 7TRANS OR A CONJUGATED TRIENE N 5CIS, N 7TRANS, N 9CIS

(30) Priorité: 08.04.2008 FR 0852359
(43) Date de publication de la demande: 19.01.2011
(73) Titulaire: Lrbeva, 35170 Bruz (FR)
(72) Inventeur: MENDY, François, F-92100 Boulogne Billancourt (FR); ROGER, Loïc, F-35135 Chantepie (FR)
(74) Mandataire: Vidon Brevets & Stratégie
(86) Numéro de dépôt international: PCT/EP2009/053470
(87) Numéro de publication internationale: WO 2009/124840

(56) Documents cités:
- EP-A1- 1 175 901
- WO-A2-2005/070412
- FR-A1- 2 319 300
- US-A1- 2008 206 275
- DATABASE CAPLUS [Online] CHEMICAL ABSTRACTS SERVICE, COLUMBUS, OHIO, US; XP002528410 extrait de STN Database accession no. 2002:499372 & JP 2002 186424 A (KANEGAFUCHI CHEMICAL IND [JP]) 2 juillet 2002 (2002-07-02)
- DATABASE CAPLUS [Online] CHEMICAL ABSTRACTS SERVICE, COLUMBUS, OHIO, US; XP002528411 extrait de STN Database accession no. 2002:503813 & JP 2002 188096 A (KANEGAFUCHI CHEMICAL IND [JP]) 5 juillet 2002 (2002-07-05)
- ANONYMOUS: "L?obésité de l'enfant, un problème de poids." CONGRES FNAMN, [Online] 21 octobre 2005 (2005-10-21), pages 1-11, XP002528474 Extrait de l'Internet: URL:http://www.smn-aquitaine.org/accueil/r esumescongres.htm> [extrait le 2009-05-18]

## Description

La présente invention concerne essentiellement une composition comprenant de l'acide myristique, et des acides gras comprenant un diène conjugué n-5cis, n-7trans ou un triène conjugué n-5cis, n-7trans, n-9cis et/ou au moins un de leurs sels. La présente invention concerne également l'utilisation de cette composition comprenant un diène conjugué n-5cis, n-7trans ou un triène conjugué n-5cis, n-7trans, n-9cis et/ou au moins un de leurs sels pour la résolution du Syndrome Inflammatoire Chronique Systémique de bas grade, nommé également syndrome inflammatoire latent.

L'inflammation est un moyen efficace, physiologique de réponse à divers types d'agressions physiques (comme le chaud, le froid, les radiations ionisantes, les rayonnements ultraviolets), chimiques, infectieuses (due à des organismes vivants pathogènes tels que les virus, bactéries, champignons). L'inflammation tend à limiter et à réparer les effets de l'agression. Cette réponse fait intervenir des phénomènes d'immunité, c'est-à-dire de résistance aux agressions. L'inflammation participe ainsi de manière importante aux défenses de l'individu.

Toutefois, l'inflammation peut également contribuer à la physiopathologie de nombreuses maladies chroniques, telles que l'athérosclérose, la surcharge pondérale, l'obésité, le syndrome métabolique, les diabètes, les maladies cardiovasculaires, sous forme d'un Syndrome Inflammatoire Chronique Systémique de bas grade (ou Latent).

Ce Syndrome Inflammatoire Chronique Systémique de bas grade, nommé également latent, résulte de la résolution partielle, non totale, de la phase aiguë du syndrome inflammatoire, le « SIRS » (Systemic Inflammatory Response Syndrome). Le SIRS s'accompagne d'une libération de médiateurs pro-inflammatoires et d'une activation des cellules immunocompétentes. Afin de contre balancer les effets du SIRS, celui-ci est suivi d'une réponse anti-inflammatoire compensatoire nommée « CARS » (Compensatory Anti-Inflammatory Response Syndrome). Lorsque cette réponse anti-inflammatoire compensatoire n'est pas parfaite, il subsiste un Syndrome Inflammatoire Chronique Systémique de bas grade, ou Latent. Ce phénomène a également été décrit comme une « dysjonction moléculaire de la réponse inflammatoire » (C.V. Rothlin et al., Cell, 2007, 131, pages 1124-1136). Le Syndrome Inflammatoire Chronique Systémique de bas grade se caractérise comme une inflammation persistante. Ce Syndrome, qui est susceptible de s'auto-entretenir, a des effets néfastes et peut entraîner la destruction de tissus, le dysfonctionnement d'organes et contribue à la physiopathologie de nombreuses maladies chroniques. Ce Syndrome Inflammatoire Chronique Systémique de bas grade a été observé aussi bien chez de jeunes patients atteints de syndrome métabolique (Warnberg J.,Marcos A., Current Opinion in Lipidology ,Janvier 2008 19 (1):11-15) que chez des personnes âgées, en gérontologie («Living Well to 100: Is Inflammation Central to Aging", Nutrition Reviews Décembre 2007, 12, partie 2, S140-S259).

Le Syndrome Inflammatoire Chronique Systémique de bas grade peut être détecté par des méthodes simples telles que le dosage de la CRP ultrasensible (protéine C-réactive dite « ultrasensible »), de l'orosomucoïde (glycoprotéine du plasma sanguin synthétisée par le foie et faisant partie du groupe des globulines), ou de la myéloperoxydase, tel que décrit dans les articles de J. Blacher et al. (J. des Maladies Vasculaires 2002,27), de G. Torre-Amione et al . (The Lancet 19 Janvier 2008, 228-236), de Warnberg J.,Marcos A. (Current Opinion in Lipidology ,Janvier 2008 19 (1):11-15) et l'article parue dans «Nutrition Reviews» («Living Well to 100: Is Inflammation Central to Aging", Décembre 2007, 12, partie 2, S140-S259).

Il semble que l'initiation (« priming ») de l'inflammation se situe au niveau des polynucléaires. L'étude des effets sur l'initiation de l'inflammation au niveau des polynucléaires devrait ainsi permettre d'étudier le Syndrome Inflammatoire Systémique de bas grade (Pham My-Chan Dang et al. J. of Clinical Investigation, 2006, 116, 7, pages 2033-2043).

Il est donc important de développer des produits permettant la résolution du Syndrome Inflammatoire Chronique Systémique de bas grade (latent).

On connaît dans l'art antérieur, aussi bien dans le domaine pharmaceutique que dans le domaine de la nutrition, plusieurs tentatives de mise au point ou d'utilisation de protocoles anti-inflammatoires pour tenter de résoudre le Syndrome Inflammatoire Chronique Systémique de bas grade.

Ainsi, dans le domaine pharmaceutique, les premiers produits ont été développés à base de stéroïdes. Toutefois, bien que l'utilisation des stéroïdes soit très efficace sur le plan aigu, elle pose beaucoup de problèmes sur le long terme. Pour cette raison les stéroïdes ont été remplacés par des anti-inflammatoires non stéroïdiens tels que les Anti-Cox-1 (anti-cyclooxygénase 1) et les Anti-Cox-2. Toutefois, il a été mis en évidence que les Anti-Cox-1 pouvaient être responsables de pathologies gastro-intestinales parfois graves et les Anti-Cox-2 ont été retirés du commerce du fait de leur implication dans le développement de pathologies cardio-vasculaires. On connaît également dans l'art antérieur, l'administration d'immunoglobines par voie intra-veineuse (« l'Immuno-adsorption therapy ») ou encore des méthodes tentant d'agir sur le TNF (Facteur de Nécrose Tumorale, « Tumor Necrosis Factor »). Cependant, la caractéristique de l'ensemble de ces traitements est d'avoir un coût élevé, et d'être beaucoup mieux adaptés au traitement du Syndrome Inflammatoire Aigu qu'au traitement du Syndrome Inflammatoire Chronique Systémique de bas grade. En effet, l'utilisation chronique de ces traitements peut s'avérer dangereuse pour la santé.

Pour une utilisation sur une longue durée, afin de traiter le Syndrome Inflammatoire Chronique Systémique de bas grade, on connaît également des méthodes basées sur l'administration de l'aspirine à faible dose. Cependant, même à faible dose, l'aspirine n'est pas totalement dépourvue d'inconvénients et d'effets secondaires indésirables tels que des gastrites ou encore des phénomènes allergiques. En outre, une étude a montré que 28% des sujets testés présentent une résistance à l'aspirine (Krasopoulos G. et al. « Aspirin resistance and risk of cardiovascular morbidity, systematic review and meta-analysis" BMJ. 2008,17 Janvier).

Dans le domaine de la nutrition, des tentatives de résolution du Syndrome Inflammatoire Chronique Systémique de bas grade (Latent) ont été très développées, en modulant les équilibres d'acides gras insaturés en oméga6/oméga3. Toutefois, ces méthodes se sont révélées inefficaces, tel que décrit dans l'article de Fritsche K. (Lipids, 2007, 42:961-979). De plus, très récemment, les inventeurs ont pu montrer que, très tôt à partir de la synthèse physiologique, à partir de l'acide alpha-linolénique, de doses considérées comme physiologiques (200 à 300 mg/jour), d'EPA (l'acide eicosapentaénoïque : acide gras insaturé en oméga 3) et de DHA (l'acide docosahéxaénoïque : acide gras insaturé en oméga 3), il apparaissait des phospholipides oxydés particulièrement compétents pour entretenir un Syndrome Inflammatoire Chronique Systémique de bas grade (Latent) (étude à paraître).

Cependant, il a été montré par S. Serhan en 2005, qu'en présence d'aspirine en faible quantité, à condition qu'il y ait une dose suffisante d'un eicosanoide insaturé en omega6 (la PGD2), l'organisme humain synthétise à partir de l'EPA membranaire, des résolvines, et à partir du DHA (en l'absence d'aspirine), des neuroprotectines, qui pourraient concourir à la résolution du Syndrome Inflammatoire Chronique Systémique de bas grade. Toutefois, cette méthode requiert l'administration d'aspirine qui présente des effets secondaires indésirables, comme exposé ci-dessus, notamment lors d'une utilisation chronique. En outre, cette méthode requiert une composition parfaitement équilibrée des membranes en acides gras insaturés en oméga 6 et en acides gras insaturés en oméga 3.

Dans le domaine de la nutrition, la Demande Internationale WO 2005/070412 décrit également des formulations à base d'acide punicique ayant un effet anti-inflammatoire. Toutefois, de part les doses d'acide punicique utilisées et l'effet anti-inflammatoire recherché, cette demande ne vise pas à obtenir la résolution du Syndrome Inflammatoire Chronique Systémique de bas grade ou Latent, devant aboutir à un équilibre physiologique, sans engendrer un autre déséquilibre.

En effet, selon les inventeurs, tous les acides gras présentant une double liaison en n-9cis (la double liaison est en position 9, à partir du groupe méthyle de l'acide gras) se situeraient dans un système concurrentiel pour la synthèse de dérivés actifs hautement insaturés en C20, C22, qui se fixeraient au niveau des phospholipides membranaires. Une fois libérés, notamment, par la phospholipase A2, ces dérivés joueraient leur rôle à des doses micro ou nanomolaires. Ainsi, l'acide oléique, l'acide ruménique, l'acide punicique, l'acide linoléique et l'acide alpha-linoléique seraient désaturés et allongés en acides gras actifs hautement insaturés en C20, C22. L'objectif du traitement du Syndrome Inflammatoire Chronique Systémique de bas grade serait alors d'obtenir un équilibre de tous ces dérivés actifs hautement insaturés, et non la suprématie d'une lignée de dérivés sur l'autre qui réenclencherait un nouveau déséquilibre. En outre, *in vivo,* la désaturation en delta 9 (la double liaison se situe en position 9 à partir du groupe carboxyle de l'acide gras) de l'acide vaccénique en acide ruménique et de l'acide gras de formule C18 :2 n-5cis,n-7trans en acide punicique est de plus très régulée sur le plan physiologique. En conséquence, l'utilisation de doses pharmacologiques pour la résolution du Syndrome Inflammatoire Chronique Systémique de bas grade (Latent) n'est pas pertinente, puisqu'elle ne permettrait pas d'obtenir et de maintenir un équilibre physiologique.

Il subsiste donc un besoin pour des produits permettant la résolution du Syndrome Inflammatoire Chronique Systémique de bas grade (ou Latent) présentant des propriétés améliorées notamment en terme de toxicité, en particulier des compositions nutraceutiques, utilisables sur une longue durée, à des doses permettant d'obtenir et de maintenir un équilibre physiologique.

De manière surprenante, les inventeurs ont établi que l'administration d'acides gras comprenant un diène conjugué n-5cis, n-7trans ou un triène conjugué n-5cis, n-7trans, n-9cis et/ou au moins un de leurs sels avait un effet sur la résolution du Syndrome Inflammatoire Chronique Systémique de bas grade (latent).

De plus, les inventeurs ont établi qu'une composition comprenant de l'acide myristique, et des acides gras comprenant un diène conjugué n-5cis, n-7trans ou un triène conjugué n-5cis, n-7trans, n-9cis et/ou au moins un de leurs sels, selon des rapports particuliers, avait un effet synergique sur la résolution du Syndrome Inflammatoire Chronique Systémique de bas grade (latent) et sur le retour à la normale de l'équilibre des réponses pro- et anti-inflammatoires (SIRS et CARS).

Ainsi selon un premier aspect, l'invention a pour objet une composition comprenant:
- de l'acide myristique ; et
- entre 10 et 70 %, de préférence entre 30 et 65 % et tout préférentiellement entre 50 et 60 % en poids par rapport au poids de l'acide myristique, d'un acide gras X1 et/ou d'au moins un de ses sels, ledit acide gras X1 comprenant de 16 à 18 atomes de carbone et un diène conjugué n-5cis, n-7trans ; et/ou
- entre 10 et 70 % de préférence entre 30 et 65 % et tout préférentiellement entre 50 et 60 % en poids par rapport au poids de l'acide myristique, d'un acide gras X2 et/ou d'au moins un de ses sels, ledit acide gras X2 comprenant de 18 à 22 atomes de carbone et un triène conjugué n-5cis, n-7trans, n-9cis.

Les compositions selon l'invention présentent notamment l'avantage de pouvoir être utilisées de manière continue en ne présentant pas d'effets secondaires indésirables, notamment en terme de toxicité.

Selon, les inventeurs, *in vivo,* l'acide gras X1 serait désaturé et éventuellement allongé en acide gras X2. L'acide myristique favoriserait la désaturation de l'acide gras X2, ce qui augmenterait la synthèse de dérivés actifs hautement insaturés en C20, C22, qui se fixeraient au niveau de phospholipides membranaires. Une fois libérés, notamment, par la phospholipase A2, ces dérivés joueraient leur rôle sur la résolution du Syndrome Inflammatoire Chronique systémique de bas grade.

On entend par « acide myristique », au sens de la présente invention, l'acide gras de formule C14 :0, équivalente à la formule suivante :

CH3(CH2)12COOH

On entend par « un acide gras comprenant un diène conjugué n-5cis, n-7trans », selon la présente invention, un acide gras comprenant une première double liaison (n-5cis) en position 5 à partir du groupe méthyle dudit acide gras et une seconde double liaison (n-7trans) en position 7 à partir du groupe méthyle dudit acide gras.

Ainsi lorsque l'acide gras X1 comprend 16 atomes de carbone et un diène conjugué n-5cis, n-7trans, ledit acide gras répond à la formule C16 :2 n-5cis, n-7trans, équivalente à la formule suivante :

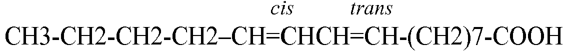

En particulier, l'acide gras X1 peut être l'acide gras de formule C16 :2 n-5cis, n-7trans ou l'acide gras de formule C18 :2 n-5cis, n-7trans.

La présence de ces deux acides gras a notamment été observée par les inventeurs dans de l'huile de beurre fractionnée par cristallisation à froid. Ces deux acides gras après désaturation en delta 9 cis (la double liaison se situe en position 9 à partir du groupe carboxyle des acides gras) et élongation en C18 pour l'acide gras en C16, donnent l'acide punicique de formule C18:2 n-5cis, n-7trans, n-9cis. Selon les inventeurs, l'acide myristique favoriserait la désaturation de l'acide punicique en delta 5 cis, delta 8 cis (via la delta 6 désaturase) qui par élongation conduirait à l'acide gras de formule C20 :5 delta 5 cis, delta 8 cis, delta 11 cis, delta 13 trans, delta 15 cis. Cet acide gras en C20, dérivé de l'acide punicique, susceptible de s'insérer dans un phospholipide membranaire et d'être libéré, notamment par la phospholipase A2, aurait une action sur la résolution du Syndrome Inflammatoire Chronique Systémique de bas grade du fait de la présence sur cet acide gras, du phénome delta 9 cis, delta 13 trans, delta 15 cis.

On entend par « un acide gras comprenant un triène conjugué n-5cis, n-7trans, n-9cis », selon la présente invention, un acide gras comprenant une première double liaison (n-5cis) en position 5 à partir du groupe méthyle dudit acide gras, une seconde double liaison (n-7trans) en position 7 à partir du groupe méthyle dudit acide gras et une troisième double liaison (n-9cis) à partir du groupe méthyle dudit acide gras.

Ainsi lorsque l'acide gras X2 comprend 18 atomes de carbone et un triène conjugué n-5cis, n-7trans, n-9cis ledit acide gras est l'acide punicique qui répond à la formule C18 :3 n-5cis, n-7trans, n-9cis, équivalente à la formule suivante :

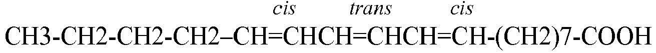

Les acides gras X2 peuvent être d'origine synthétique ou naturelle.

On entend par « sel d'acide gras », au sens de la présente invention, une molécule dans laquelle l'atome H du groupe carboxyle -COOH d'un acide gras est remplacé par un autre atome tel que sodium, potassium, magnésium ou une autre molécule tel qu'un acide aminé.

Les sels d'acides gras X1 et X2 utilisables dans les compositions selon l'invention sont l'ensemble des sels d'acides gras qui ne sont pas toxiques, notamment pour les êtres humains et les animaux. Les sels d'acide gras X1 et X2 peuvent être d'origine synthétique ou naturelle.

En particulier, les sels d'acides gras X1 et X2 peuvent être choisis dans le groupe comprenant les sels de métaux alcalins, les sels de métaux alcalino-terreux et les sels d'acides aminés, de préférence les sels d'acides aminés et tout préférentiellement les sels d'arginine et de lysine.

Ainsi, lesdits sels d'acides gras X1 et X2 peuvent être des sels d'acides aminés d'acides gras X1 et X2 dans lesquels l'atome H du groupe carboxyle -COOH de l'acide gras est remplacé par un acide aminé tel que la glycine, l'alanine, la valine, la leucine, l'isoleucine, l'acide aspartique, l'asparagine, l'acide glutamique, la glutamine, la sérine, la thréonine, la cystéine, la méthionine, la lysine, l'arginine, la phénylalanine, la tyrosine, le tryptophane, l'histidine, la proline, la cystine, l'ornithine.

De préférence, les sels d'acides gras X1 et X2 inclus dans les compositions selon l'invention sont des sels d'arginine et/ou de lysine. Ces sels d'arginine et de lysine présentent l'avantage d'être solubles dans l'eau, ce qui facilite leur administration.

À titre d'exemple, le sel d'arginine de l'acide punicique, répond à la formule suivante :

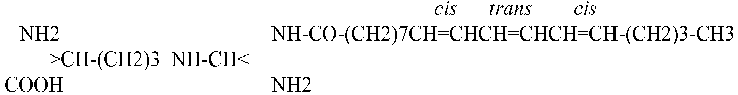

Lesdits sels d'acides gras X1 et X2 peuvent être obtenus par des techniques bien connus de l'Homme du Métier.

En particulier, dans la composition selon l'invention au moins une partie de l'acide myristique compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 %, et tout préférentiellement au moins 70 % de l'acide myristique estérifié à des chaînes de glycérol est en position sn-2.

On entend par « au moins une partie d'un acide gras (tel que l'acide myristique) compris dans la composition », au moins une molécule d'un acide gras comprise dans la composition. Ceci peut varier d'une molécule d'acide gras à l'ensemble des molécules d'acide gras comprises dans la composition.

On entend par « triacylglycérol », au sens de la présente invention, un glycéride dans lequel le glycérol est estérifié par trois acides gras. Les triacylglycérols répondent à la formule suivante: RCOO-CH2CH(-OOCR')CH2-OOCR", dans laquelle chaque RCO, R'CO et R"CO représente le groupe acyle d'un acide gras. Les trois acides gras RCOOH, R'COOH et R"COOH qui estérifient le glycérol peuvent être identiques ou différents.

Dans cette formule RCOO-CH2CH(-OOCR')CH2-OOCR" :
- l'acide gras RCOOH qui estérifie le glycérol, est en position sn-1 du triacylglycérol, ou l'acide gras RCOOH est estérifié à une chaîne de glycérol dans la configuration stéréochimique sn-1 ;
- l'acide gras R'COOH qui estérifie le glycérol, est en position sn-2 du triacylglycérol ou l'acide gras R'COOH est estérifié à une chaîne de glycérol dans la configuration stéréochimique sn-2 ;
- l'acide gras R"COOH qui estérifie le glycérol, est en position sn-3 du triacylglycérol, ou l'acide gras R"COOH est estérifié à une chaîne de glycérol dans la configuration stéréochimique sn-3.

Ainsi, on entend, au sens de la présente invention par « l'acide myristique estérifié à une chaîne de glycérol dans la configuration stéréochimique sn-1,2,3 », un triacylglycérol comprenant de l'acide myristique en position sn-1 et sn-2 et sn-3.

Ainsi, on entend par « la position sn-2 » d'un triacylglycérol, la position de l'acide gras R'COOH qui estérifie le glycérol, dans la formule du triacylglycérol RCOO-CH2CH(-OOCR')CH2-OOCR".

Ainsi, un triacylglycérol comprenant de l'acide myristique en position sn-2 peut être représenté par la formule suivante :

RCOO-CH2CH(-OOCR')CH2-OOCR"

dans laquelle RCO et R"CO représentent le groupe acyle d'un acide gras, R et R" pouvant être identiques ou différents; et
R' est le groupe CH3(CH2)12 ;
R'CO étant le groupe acyle de l'acide myristique ;
les acides gras RCOOH et R"COOH qui estérifient le glycérol pouvant être tout type d'acide gras, notamment de l'acide myristique, et pouvant être issus d'au moins une matière grasse d'origine animale, notamment lactique et/ou d'origine végétale.

On entend par « au moins 50 % de l'acide myristique estérifié à des chaînes de glycérol est en position sn-2 », le fait qu'au moins 50 % de l'acide myristique qui est compris dans les triacylglycérols, est en position sn-2 desdits triacylglycérols.

Lesdits triacylglycérols comprenant de l'acide myristique peuvent comprendre en outre tout type d'acide gras saturés, mono-insaturés, polyinsaturés, de préférence l'acide oléique, l'acide palmitique, l'acide laurique, l'acide caprylique, l'acide caprique, l'acide butyrique, lesdits acides gras et/ou lesdits triacylglycérols pouvant notamment être issus d'au moins une matière grasse d'origine animale, notamment lactique et/ou d'origine végétale.

En particulier, lesdits triacylglycérols comprenant de l'acide myristique peuvent comprendre en outre, notamment en position sn-3, un phosphate lié par une liaison ester au groupe hydroxyle d'une sérine, d'une éthanolamine et/ou d'une choline donnant un phosphoglycéride.

Les acides gras (tel que l'acide myristique) estérifiés à des chaînes de glycérol en position sn-2 présentent l'avantage (contrairement aux acides gras en position sn-1) de ne pas être libérés par la lipase pancréatique et de ne pas être béta-oxydés.

En particulier, dans la composition selon l'invention, au moins une partie de l'acide gras X1 compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 %, et tout préférentiellement au moins 70 % de l'acide gras X1 estérifié à des chaînes de glycérol est en position sn-2.

On entend par « au moins 50 % de l'acide gras X1 estérifié à des chaînes de glycérol est en position sn-2 », le fait qu'au moins 50 % de l'acide gras X1 qui est compris dans les triacylglycérols, est en position sn-2 desdits triacylglycérols.

Lesdits triacylglycérols comprenant de l'acide gras X1 peuvent comprendre en outre tout type d'acide gras saturés, mono-insaturés, polyinsaturés, de préférence l'acide oléique, l'acide palmitique, l'acide laurique, l'acide caprylique, l'acide caprique, l'acide butyrique, l'acide alpha-linolénique, lesdits acides gras et/ou lesdits triacylglycérols pouvant notamment être issus d'au moins une matière grasse d'origine animale, notamment lactique et/ou d'origine végétale.

En particulier, lesdits triacylglycérols comprenant de l'acide gras X1 peuvent comprendre en outre, notamment en position sn-3, un phosphate lié par une liaison ester au groupe hydroxyle d'une sérine, d'une éthanolamine et/ou d'une choline donnant un phosphoglycéride.

L'acide myristique et les acides gras X1 peuvent être d'origine synthétique ou naturelle.

Selon un mode de réalisation particulier de la composition selon l'invention, au moins une partie de l'acide myristique compris dans la composition, et/ou au moins une partie de l'acide gras X1 compris dans la composition, sont issues d'au moins une matière grasse d'origine animale, notamment de matière grasse lactique, et/ou d'origine végétale.

On entend par « matière grasse d'origine animale », au sens de la présente invention, tout corps gras issu d'au moins un animal. Lesdits animaux peuvent être notamment des poissons ou des mammifères marins.

On entend par « matière grasse d'origine végétale », au sens de la présente invention, tout corps gras issu d'au moins un végétal y compris champignons et algues.

On entend par « matière grasse lactique », au sens de la présente invention, toute matière grasse d'origine laitière, notamment du lait de vache, de jument, d'ânesse, de brebis, de chèvre, de bufflonne.

L'acide myristique issu de matière grasse lactique présente l'avantage qu'au moins 50 % de l'acide myristique estérifié à des chaînes de glycérol est en position sn-2.

Les procédés permettant d'obtenir l'acide myristique et/ou l'acide gras X1, à partir de matière grasse d'origine animale, notamment de matière grasse lactique, et/ou d'origine végétale peuvent être tout type de procédés classiquement utilisés par l'Homme du Métier tels que décrit dans l'ouvrage « Dry fractionation and selectivity : Innovations technologiques » (DEFFENSE E., OCL. Oléagineux, corps gras, lipides, 1998, vol. 5, n°5, pp. 391-395). À titre d'exemples de tels procédés, on peut citer des procédés physiques tels que la distillation moléculaire sous vide, l'extraction au gaz supercritique et la cristallisation par voie sèche.

En particulier, la matière grasse d'origine végétale est susceptible d'être obtenus par extraction par un solvant notamment organique, par extraction au soxhlet, par pression mécanique à froid, notamment à l'aide d'une presse à vis continue, qui sont des technologies bien connues de l'Homme du Métier.

La matière grasse peut être utilisée brut ou raffinée. On entend par « raffinage » au sens de la présente invention, les opérations unitaires de purification des lipides d'origine végétale bien connues de l'Homme du Métier, parmi lesquelles on peut citer la démucilagination, la désacidification, la décoloration, la désodorisation et la frigélisation.

En particulier, l'acide myristique et/ou l'acide gras X1 peuvent être obtenus par cristallisation fractionnée de matière grasse, en particulier d'origine animale, notamment de matière grasse lactique, et/ou d'origine végétale. Ce procédé présente l'avantage de concentrer les triacylglycérols comprenant de l'acide myristique et lesdits triacylglycérols comprenant de l'acide gras X1 présents dans la matière grasse.

En particulier, dans la composition selon l'invention, au moins une partie de l'acide gras X2 compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 %, et tout préférentiellement au moins 70 % de l'acide gras X2 estérifié à des chaînes de glycérol est en position sn-2.

On entend par « au moins 50 % de l'acide gras X2 estérifié à des chaînes de glycérol est en position sn-2 », le fait qu'au moins 50 % de l'acide gras X2 qui est compris dans les triacylglycérols, est en position sn-2 desdits triacylglycérols.

Lesdits triacylglycérols comprenant de l'acide gras X2 peuvent comprendre, en outre, tout type d'acide gras saturés, mono-insaturés, polyinsaturés, de préférence l'acide oléique, l'acide palmitique, l'acide laurique, l'acide caprylique, l'acide caprique, l'acide butyrique, l'acide alpha-linolénique, lesdits acides gras et/ou lesdits triacylglycérols pouvant notamment être issus d'au moins une matière grasse d'origine animale, notamment lactique et/ou d'origine végétale.

En particulier, lesdits triacylglycérols comprenant de l'acide gras X2 peuvent comprendre en outre, notamment en position sn-3, un phosphate lié par une liaison ester au groupe hydroxyle d'une sérine, d'une éthanolamine et/ou d'une choline donnant un phosphoglycéride.

Selon un mode de réalisation particulier de la composition selon l'invention, la quantité d'acide myristique dans ladite composition correspond à au moins 1,2 %, de préférence de 1,2 à 1,8 % et tout préférentiellement à 1,5 % de l'apport énergétique total journalier recommandé pour un être humain.

On entend par « apport énergétique total journalier recommandé » la quantité totale d'énergie qui est recommandée d'apporter à l'organisme, par jour. L'apport énergétique total journalier recommandé pour un être humain peut notamment être compris entre 1800 et 2500 Kcalories, de préférence entre 2000 et 2400 Kcalories et tout préférentiellement 2200 Kcalories dont en particulier, entre 25 et 40 %, de préférence entre 30 et 37 % et tout préférentiellement 33 % de l'énergie provient de lipides.

L'apport énergétique total journalier recommandé pour un être humain a été exprimé selon la présente invention pour un sujet adulte ayant un poids compris entre 50 et 80 kilos.

Il faut noter que l'apport énergétique total journalier effectif d'un sujet peut être différent (moins ou plus élevé, notamment pour des raisons médicales) de l'apport énergétique total journalier recommandé.

On entend par « Kcalories » ou Kilocaries, au sens de la présente invention l'unité de mesure de la chaleur utilisée pour fixer la valeur nutritive des aliments par estimation de la chaleur dégagée par leur oxydation dans l'organisme. Une Kilocalorie représente la quantité de chaleur nécessaire pour élever de 0 à 1 °C 1 kg d'eau liquide. Une Kilocalorie équivaut à 4,18 Kilojoules.

On entend par « lipide », au sens de la présente invention, des triacylglycérols, des phospholipides, des sphingolipides et tout acide gras ou dérivé (tel que les esters, les aldéhydes) d'un acide gras.

Selon un mode de réalisation particulier de la composition selon l'invention, la quantité d'acide myristique dans ladite composition est comprise 1,6 et 8,8 grammes, de préférence entre 1,9 et 6,4 grammes et tout préférentiellement entre 2,9 et 4,3 grammes.

La quantité d'acide myristique dans la composition selon l'invention présente l'avantage que l'acide myristique est à une teneur permettant d'augmenter le taux de lipoprotéines de haute densité (« HDL », "High Density lipoprotein") sans augmenter le taux de lipoprotéines de basse densité (LDL, « Low density Lipoprotein »), améliorant ainsi le rapport lipoprotéines de basse densité/lipoprotéines de haute densité, diminuant aussi le taux plasmatique de la lipoprotéine(a) (Lp(a))(réduisant ainsi son effet athéro-thrombogène).

Les lipoprotéines de haute densité (HDL) sont responsables du transport du cholestérol vers le foie, où il pourra être éliminé. Cette fonction permet d'éviter l'accumulation de cholestérol dans les vaisseaux sanguins et donc d'éviter, notamment, les risques d'athérosclérose. Les lipoprotéines de basse densité (LDL) transportent le cholestérol, libre ou estérifié, dans le sang et à travers le corps pour les apporter aux cellules. Un défaut de captation des LDL par les cellules des tissus augmente le taux de cholestérol dans les vaisseaux sanguins et en s'y déposant entraîne l'athérosclérose.

Selon un mode de réalisation particulier de la composition selon l'invention, la dose journalière d'acide myristique est comprise entre 2 et 11 %, de préférence entre 2,4 et 8 % et tout préférentiellement entre 3,6 et 5,4 % de la consommation journalière totale de lipides d'un sujet.

On entend par « dose journalière », au sens de la présente invention, la quantité d'une substance ou d'une composition administrée quotidiennement, ou un produit comprenant la quantité d'une substance ou d'une composition administrée quotidiennement. La dose journalière a été exprimée selon la présente invention pour un sujet adulte, notamment un être humain adulte, ayant un poids compris entre 50 et 80 kilos.

On entend par « consommation journalière totale de lipides d'un sujet », au sens de la présente invention, la quantité totale de lipides consommés par un sujet quotidiennement.

La consommation journalière totale de lipides d'un sujet peut être comprise entre 60 et 100 grammes, de préférence entre 70 et 90 grammes et tout préférentiellement 80 grammes. La dose consommation journalière totale de lipides d'un sujet a été exprimée selon la présente invention pour un sujet adulte, notamment un être humain adulte, ayant un poids compris entre 50 et 80 kilos.

En particulier, la quantité d'acide gras X1 et/ou d'au moins un de ses sels dans ladite composition correspond à au moins 0,15 %, de préférence à 0,3 à 0,9 % et tout préférentiellement à 0,4 à 0,8 % de l'apport énergétique total journalier recommandé pour un être humain.

Selon un mode de réalisation particulier de la composition selon l'invention, la quantité d'acide gras X1 et/ou d'au moins un de ses sels dans ladite composition est comprise entre 0,4 et 4 grammes, de préférence entre 0,6 et 3 grammes et tout préférentiellement entre 1 et 2 grammes.

Selon un mode de réalisation particulier de la composition selon l'invention, la dose journalière d'acide gras X1 et/ou d'au moins un de ses sels est comprise entre 0,6 et 5 %, de préférence entre 0,8 et 4 % et tout préférentiellement entre 1,25 et 2,5 % de la consommation journalière totale de lipides d'un sujet.

En particulier, la quantité d'acide gras X2 et/ou d'au moins un de ses sels dans ladite composition correspond à au moins 0,3 %, de préférence à 0,3 à 0,9 % et tout préférentiellement à 0,4 à 0,8 % de l'apport énergétique total journalier recommandé pour un être humain.

Selon un mode de réalisation particulier de la composition selon l'invention, la quantité d'acide gras X2 et/ou d'au moins un de ses sels dans ladite composition est comprise 0,5 et 4 grammes, de préférence entre 0,6 et 3 grammes et tout préférentiellement entre 1 et 2 grammes.

Selon un mode de réalisation particulier de la composition selon l'invention, la dose journalière d'acide gras X2 et/ou d'au moins un de ses sels est comprise entre 0,6 et 5 %, de préférence entre 0,8 et 4 % et tout préférentiellement entre 1,25 et 2,5 % de la consommation journalière totale de lipides d'un sujet.

Les quantités d'acide myristique et d'acides gras X1 et/ou X2 et/ou leurs sels utilisées dans les compositions selon l'invention présente l'avantage d'être physiologiquement acceptable et ainsi de ne pas bouleverser les régulations physiologiques.

En outre, la quantité d'acide gras X1 et/ou X2 dans la composition selon l'invention, présente l'avantage de conserver un équilibre entre les acides gras insaturés en n-5cis, n-7trans et les acides gras insaturés en oméga 3.

En particulier, ledit acide gras X2, est l'acide punicique.

On entend par « acide punicique », au sens de la présente invention, l'acide gras comprenant 18 atomes de carbone et un triène conjugué n-5cis, n-7trans, n-9cis, répondant à la formule C18 :3 n-5cis, n-7trans, n-9cis équivalent à la formule suivante :

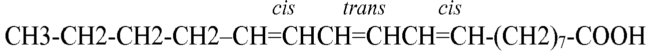

L'acide punicique est encore dénommé l'acide octadécatrienoïque delta 9-cis, delta 11-trans, delta 13-cis.

La présence d'acide punicique dans la composition selon l'invention présente l'avantage d'optimiser la résolution du Syndrome Inflammatoire Chronique systémique de bas grade.

L'acide punicique peut être d'origine synthétique ou naturelle.

En particulier, au moins une partie de l'acide punicique compris dans la composition est issue d'au moins un végétal choisi dans le groupe comprenant les végétaux de la famille des Cucurbitaceae, notamment *Trichosanthes cucumerina anguina, Momordica charantia, Cucurbita maxima, Citrullus lanatus* et les végétaux de la famille des Punicaceae, notamment *Punica granatum.*

En particulier, au moins une partie de l'acide punicique compris dans la composition peut être issu de plantes génétiquement modifiées, en particulier de *Brassica napus* génétiquement modifié pour produire de l'acide punicique, tel que décrit dans la communication de Nagasaki et al. («Plant Seed Oil Containing cis 9,trans 11, cis 13 conjugated Linolenic acid Affects Body Fate Mass and Lipid Metabolism in rodents" K. KOBA and al. Univ. of Nagasaki, Kumamoto, Tamagawa, Hokaido, Kyushu, Ohio State University Colombus, Mitsubishi, Kagaku, Bio-Clinica Labs Inc., Communication Orale, Livre des Abstracts, AOCS 2007, Québec 13616 Mai 2007).

L'obtention d'acide punicique à partir de végétaux peut être réalisée selon toutes techniques bien connues de l'Homme du Métier, telles que selon les techniques d'obtention de matière grasse végétale décrites ci-dessus.

La composition selon l'invention peut comprendre en outre :
- entre 45 et 75 % de préférence entre 50 et 70 % et tout préférentiellement entre 55 et 68 % en poids par rapport au poids de l'acide myristique, d'acide alpha-linolénique.

On entend par « acide alpha-linolénique », au sens de la présente invention, l'acide gras répondant à la formule suivante :

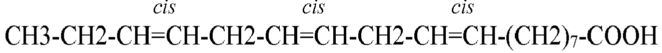

Les compositions selon l'invention comprenant en outre de l'acide alpha-linolénique présentent l'avantage d'optimiser la résolution de syndrome inflammatoire Chronique Systémique de bas grade.

En particulier, au moins une partie de l'acide alpha-linolénique compris dans la composition selon l'invention est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 % et tout préférentiellement au moins 70 % de l'acide alpha-linolénique estérifié à des chaînes de glycérol est en position sn-2.

On entend par « au moins 50 % de l'acide alpha-linolénique estérifié à des chaînes de glycérol est en position sn-2 », le fait qu'au moins 50 % de l'acide alpha-linolénique qui est compris dans les triacylglycérols, est en position sn-2 desdits triacylglycérols. Lesdits triacylglycérols comprenant de l'acide l'acide alpha-linolénique peuvent comprendre, en outre, tout type d'acide gras saturés, mono-insaturés, polyinsaturés, de préférence l'acide oléique, l'acide palmitique, l'acide laurique, l'acide caprylique, l'acide caprique, l'acide butyrique, lesdits acides gras et/ou lesdits triacylglycérols pouvant notamment être issus d'au moins une matière grasse d'origine animale, notamment lactique et/ou d'origine végétale.

En particulier, lesdits triacylglycérols comprenant de l'acide alpha-linolénique peuvent comprendre en outre, notamment en position sn-3, un phosphate lié par une liaison ester au groupe hydroxyle d'une sérine, d'une éthanolamine et/ou d'une choline donnant un phosphoglycéride.

L'acide alpha-linolénique estérifié à des chaînes de glycérol en position sn-2 présente l'avantage (contrairement aux acides gras en position sn-1) de ne pas être libéré par la lipase pancréatique et de ne pas être béta-oxydé. L'acide alpha-linolénique estérifié à des chaînes de glycérol en position sn-2 est absorbé par l'organisme sous forme de mono-glycéride et sert à la synthèse de triacylglycérols et/ou de phospholipides, rentrant ainsi dans la voie de synthèse d'EPA et de DHA.

En particulier, la quantité d'acide alpha-linolénique dans ladite composition correspond à au moins 0,7 %, de préférence à 0,7 à 1,1 % et tout préférentiellement à 0,9 % de l'apport énergétique total journalier recommandé pour un être humain.

Selon un mode de réalisation particulier de la composition selon l'invention, la quantité d'acide alpha-linolénique dans ladite composition est compris entre 1 et 4 grammes, de préférence entre 1,5 et 3 grammes et tout préférentiellement de 2 grammes.

Selon un mode de réalisation particulier de la composition selon l'invention, la dose journalière d'acide alpha-linolénique est compris entre 1,25 et 5 %, de préférence entre 1,8 et 3,7 % et tout préférentiellement de 2,5 % de la consommation journalière totale de lipides d'un sujet.

L'acide alpha-linolénique peut être d'origine synthétique ou naturelle.

En particulier, au moins une partie de l'acide alpha-linolénique compris dans la composition est issue d'au moins un végétal choisi dans le groupe comprenant *Brassica napus* et les végétaux du genre *Juglans,* de préférence *Brassica napus.*

Les triacylglycérols, comprenant de l'acide alpha-linolénique, issus de *Brassica napus* présentent l'avantage d'avoir un rapport d'acide alpha-linolénique en position sn-2/acide alpha-linolénique en position sn-1 supérieur à 1 et un rapport acide alpha-linolénique/acide linolénique intéressant.

En effet, les triacylglycérols, comprenant de l'acide alpha-linolénique, issus de *Brassica napus* présentent l'avantage qu'au moins 60 % de l'acide alpha-linolénique estérifié à des chaînes de glycérol est en position sn-2.

L'obtention d'acide alpha-linolénique à partir de végétaux peut être réalisée selon toutes techniques bien connues de l'Homme du Métier, telles que selon les techniques d'obtention de matière grasse végétale décrites ci-dessus.

La composition selon l'invention peut comprendre en outre :
- entre 15 et 70 %, de préférence entre 25 et 40 % et tout préférentiellement entre 30 et 35 % en poids par rapport au poids de l'acide myristique, d'acide stéaridonique.

On entend par « acide stéaridonique », au sens de la présente invention, l'acide gras répondant à la formule suivante :

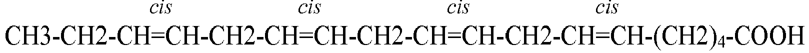

La présence d'acide stéaridonique dans les compositions selon l'invention, présentent l'avantage d'optimiser la résolution du Syndrome Inflammatoire Chronique Systémique de bas grade. En particulier, la présence d'acide stéaridonique dans les compositions selon l'invention permet de diminuer l'apport d'alpha-linolénique dont 30 à 40% restent éminemment oxydables, tout en conservant la possibilité de rentrer dans un chemin préférentiel des rôles structuraux et de la synthèse de l'EPA, du DHA et de leurs dérivés, sans contourner la régulation de la Delta-5 désaturase comme le fait l'addition d'EPA et/ou de DHA.

En particulier, au moins une partie de l'acide stéaridonique compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 %, et tout préférentiellement au moins 70 % de l'acide stéaridonique estérifié à des chaînes de glycérol est en position sn-2.

On entend par « au moins 50 % de l'acide stéaridonique estérifié à des chaînes de glycérol est en position sn-2 », le fait qu'au moins 50 % de l'acide stéaridonique qui est compris dans les triacylglycérols, est en position sn-2 desdits triacylglycérols.

Lesdits triacylglycérols comprenant de l'acide stéaridonique peuvent comprendre, en outre, tout type d'acide gras saturés, mono-insaturés, polyinsaturés, de préférence l'acide oléique, l'acide palmitique, l'acide laurique, l'acide caprylique, l'acide caprique, l'acide butyrique, l'acide gamma-linolénique, lesdits acides gras et/ou lesdits triacylglycérols pouvant notamment être issus d'au moins une matière grasse d'origine animale, notamment lactique et/ou d'origine végétale.

En particulier, lesdits triacylglycérols comprenant de l'acide stéaridonique peuvent comprendre en outre, notamment en position sn-3, un phosphate lié par une liaison ester au groupe hydroxyle d'une sérine, d'une éthanolamine et/ou d'une choline donnant un phosphoglycéride.

En particulier, la quantité d'acide stéaridonique dans ladite composition correspond à au moins 0,25 %, de préférence à 0,25 à 0,9 % et tout préférentiellement à 0,3 à 0,8 % de l'apport énergétique total journalier recommandé pour un être humain.

Selon un mode de réalisation particulier de la composition selon l'invention, la quantité d'acide stéaridonique dans ladite composition est comprise entre 0,4 et 4 grammes de préférence entre 0,55 et 3,2 grammes et tout préférentiellement entre 0,8 et 2 grammes.

Selon un mode de réalisation particulier de la composition selon l'invention, la dose journalière d'acide stéaridonique est comprise entre 0,5 et 5 %, de préférence entre 0,7 et 4 % et tout préférentiellement entre 1 et 2,5 % de la consommation journalière totale de lipides d'un sujet.

Selon un mode de réalisation particulier, la composition selon l'invention constitue une dose journalière.

L'invention a également pour objet les compositions selon l'invention « consistant en » les composés décrits ci-dessus comme étant compris dans lesdites compositions.

La composition selon l'invention peut être choisie dans le groupe comprenant les compositions alimentaires, notamment diététique, les compositions pharmaceutiques et les compositions nutraceutiques.

On entend par « composition alimentaire », au sens de la présente invention tout type de composition adaptée notamment à l'alimentation, la consommation ou à la nutrition. Les compositions alimentaires selon l'invention comprennent notamment les compléments alimentaires, les compositions diététiques, les alicaments.

Les compositions alimentaires selon l'invention peuvent se présenter sous une forme choisie dans le groupe comprenant des produits pulvérulents à reconstituer, des formes alimentaires prêtes à consommer telles que boissons, potages, sauces, matières grasses composées allégées, pâtes à tartiner, desserts lacto-végétal, gélifiés, entremets, biscuits, gâteaux, viennoiseries.

Ces différentes formes peuvent être obtenues par des techniques bien connues de l'Homme du Métier.

Les compléments alimentaires selon l'invention peuvent être ajoutés à différents aliments tels que des crèmes, des boissons laitières, des potages des sauces.

On entend par « composition pharmaceutique », au sens de la présente invention tout type de composition adaptée notamment à un usage médical.

Les compositions pharmaceutiques selon l'invention peuvent se présenter sous différentes formes adaptées notamment au mode d'administration.

En particulier, les compositions pharmaceutiques selon l'invention peuvent se présenter sous une forme choisie dans le groupe comprenant les gélules, les poudres, les granulés, les solutions et suspensions orales, les sirops, les gouttes buvables, les préparations pour usage parentéral telles que des émulsions lipidiques pour un usage intraveineux.

Ces différentes formes peuvent être obtenues par des techniques bien connues de l'Homme du Métier.

On entend par « composition nutraceutique », au sens de la présente invention toute composition mise au point à partir de substances alimentaires, mais rendu disponible sous forme une forme médicinale.

Les compositions selon l'invention peuvent être administrées par différentes voies compatibles avec l'effet recherché, de préférence par voie orale ou entérale ou parentérale.

Les compositions selon l'invention, notamment alimentaires, peuvent comprendre en outre des protéines laitières et/ou végétales. Il est possible d'utiliser, seules ou en mélange toute protéine d'origine alimentaire en fonction de sa double aptitude fonctionnelle (fonction organoleptique et texturante) et nutritionnelle (équilibre en acides aminés ou apport spécifique). Les protéines peuvent être introduites sous forme entière, partiellement hydrolysées, ou fractionnées (telles que caséines, protéines sériques, ovoprotéines) enrichies ou non en l'une de ses composantes. Les compositions, notamment alimentaires, selon l'invention, peuvent être supplémentées en substances nutritionnelles telles que glucides complexes, glucides simples, fibres, vitamines, minéraux et toutes substances naturelles ou non, ayant des effets biologiques synergiques à l'activité de la composition selon l'invention telles que des acides aminés, fibres, poly phénols, caroténoïdes, stérols.

Les compositions selon l'invention peuvent comprendre en outre au moins un adjuvant choisi dans le groupe comprenant les édulcorants, de préférence acalorique, des agents donnant du goût, des colorants appropriés, des arômes et des aromates, des texturants.

L'invention a également pour objet l'utilisation d'une composition selon l'invention en tant que médicament.

Lorsque la composition selon l'invention est une composition pharmaceutique ou est utilisée en tant que médicament les sels d'acides gras X1 et X2 utilisables sont de préférence des sels pharmaceutiquement acceptables.

On entend par « sels pharmaceutiquement acceptables », au sens de la présente invention, des sels appropriés à un usage pharmaceutique. À titre d'exemples de sels pharmaceutiquement acceptables, on peut citer les sels d'arginine et les sels de lysine.

Les sels pharmaceutiquement acceptables d'acides gras X1 et X2 peuvent être obtenus par des techniques bien connues de l'Homme du Métier.

L'invention a également pour objet l'utilisation d'une composition selon l'invention pour la fabrication d'une composition alimentaire, pharmaceutique ou nutraceutique destinée à la prévention et/ou au traitement de syndrome inflammatoire chronique systémique de bas grade.

L'invention a également pour objet l'utilisation d'une composition selon l'invention pour son application en tant que composition alimentaire, pharmaceutique ou nutraceutique dans la prévention et/ou le traitement de syndrome inflammatoire chronique systémique de bas grade.

En particulier la composition selon l'invention est destinée à être administrée à des sujets présentant une surcharge pondérale et/ou à des sujets âgés et/ou à des sujets atteints d'une pathologie choisie dans le groupe comprenant l'obésité, le syndrome métabolique, l'insulinorésistance, les diabètes, l'athérosclérose, les maladies cardiovasculaires, la dégénérescence cérébrale, la maladie d'Alzheimer, les accidents vasculaires cérébraux.

L'invention a également pour objet l'utilisation d'une composition selon l'invention pour la préparation d'une composition alimentaire, pharmaceutique ou nutraceutique destinée à la prévention et/ou au traitement de pathologies et/ou de troubles pouvant être améliorés ou évités par le traitement d'un syndrome inflammatoire chronique systémique de bas grade.

L'invention a également pour objet l'utilisation d'une composition selon l'invention en tant que composition alimentaire, pharmaceutique ou nutraceutique pour la prévention et/ou le traitement de pathologies et/ou de troubles pouvant être améliorés ou évités par le traitement d'un syndrome inflammatoire chronique systémique de bas grade.

En particulier, les pathologies sont choisies dans le groupe comprenant l'obésité, le syndrome métabolique, l'insulinorésistance, les diabètes, l'athérosclérose, les maladies cardiovasculaires, la dégénérescence cérébrale, la maladie d'Alzheimer, les accidents vasculaires cérébraux.

En particulier, la dose journalière d'acide myristique est comprise 1,6 et 8,8 grammes, de préférence entre 1,9 et 6,4 grammes et tout préférentiellement entre 2,9 et 4,3 grammes.

En particulier, la dose journalière d'acide alpha-linolénique est comprise entre 1 et 4 grammes, de préférence entre 1,5 et 3 grammes et tout préférentiellement de 2 grammes.

En particulier, la dose journalière d'acide stéaridonique est comprise entre 0,4 et 4 grammes de préférence entre 0,55 et 3,2 grammes et tout préférentiellement entre 0,8 et 2 grammes.

Les doses journalières d'acides gras de la composition selon l'invention peuvent être administrées notamment en 1 à 3 prises par jour.

Les compositions selon l'invention peuvent être administrées, notamment selon ces doses journalières, pendant au moins 1 jour à toute une vie.

Les acides gras X1 et X2 sont tels que définis dans les compositions selon l'invention. En particulier, l'acide gras X2 est l'acide punicique. En effet, les inventeurs ont notamment montré que l'acide punicique, permet de bloquer l'initiation de l'inflammation, puis de diminuer le taux de myeloperoxydase et le taux de phospholipides oxydés et ainsi de résoudre le syndrome inflammatoire chronique systémique de bas grade.

Les acides gras X1 et/ou X2 peuvent être estérifiés à des chaînes de glycérol comme décrits pour les compositions selon l'invention.

Les sels d'acides gras X1 et X2 peuvent être les sels compris dans les compositions selon l'invention.

D'autres avantages et caractéristiques de l'invention apparaîtront au regard des exemples qui suivent.

Ces exemples sont donnés à titre illustratif et non limitatif.

### Exemples

### I. Exemple 1 : Mise en évidence de l'effet sur le taux de phospholipides oxydés et de la lipoprotéine Lp(a) de compositions selon l'invention

L'étude qui s'est déroulée pendant 1 an portait sur 29 moines. Les sujets étaient âgés de 25 à 85 ans (moyenne de 57 ans) et pesaient entre 53 et 99 kilos (moyenne de 71 kilos). Au cours de cette étude deux régimes expérimentaux ont été testés pendant une période de 3 mois chacun, suivie d'une période de 3 mois de non-régime (régime contrôle). Chaque régime (régime 1 et 2 et régime contrôle) apportait 2100 Kilocalories par jour. Les régimes 1 et 2 apportaient 2,1 grammes d'acide alpha-linolénique par jour. Le régime 1 apportait 2,9 grammes d'acide myristique par jour. Le régime 2 apportait 4 grammes d'acide myristique par jour, issu de matière grasse laitière fractionnée, et 0,4 grammes d'acide gras de formule C18 :2 n-5cis, n-7trans.

Le test Wilcoxon a été utilisé pour comparer les taux de phospholipides oxydés et de lipoprotéines dans le plasma. Les taux sont exprimés en mg/dl (avec p<0.01).

Les résultats obtenus sont les suivants :

| | Régime 1 | Régime 2 |
|---|---|---|
| Phospholipides Oxydés | 27.6+/-5.9 | 24.7+/-7.2 |
| Phospholipides Oxydés/ apolipoprotéine B | 30.0+/-6.9 | 26.2+/-7.5 |
| Lp(a) lipoprotéine | 21.0+/-19 | 15.0+/-12 |

L'administration à des doses journalières particulières d'acide myristique combiné avec l'acide gras de formule C18:2 n-5cis, n-7trans permet d'obtenir une baisse significative du taux de phospholipides oxydés et de lipoprotéine Lp(a) dans le plasma.

Or le rôle du taux plasmatique des phospholipides oxydés et de la lipoprotéine Lp(a) dans l'inflammation a été mis en évidence par plusieurs équipes tels que décrits dans les articles de B. Gabriel Gugiu et al. (« Protein targets of oxidized phospholipids in endothelial cells » J. Lipid Res. Mars 2008,49,510-520) et de JunJun Wang et al. (« Native,oxidized lipoprotein(a) and lipoprotein(a) immune complex in patients with active and inactive rheumatoid arthritis ;-Plasma concentrations and relationship to inflammation » Clinica chimica acta 2008,390,67-71).

### Corrélation entre Phospholipides oxydés et lipoprotéine Lp(a)

| | Phospholipides Oxydés/apolipoprotéine B | lipoprotéine Lp(a) |
|---|---|---|
| Régime 1 | | |
| Phospholipides Oxydés | r=0,65 p<0,001 | r=0,5 p<0,02 |
| Phospholipides | / | r=0,5 p<0,02 |
| Oxydés/apolipoprotéine B | | |
| Régime 2 | | |
| Phospholipides Oxydés | r=0,89 p<0,0001 | NS |
| Phospholipides | / | NS |
| Oxydés/apolipoprotéine B | | |

Avec le régime 1, les Phospholipides Oxydés sont corrélés positivement avec le ratio Phospholipides Oxydés/apolipoprotéine B-100 (r=0,65 p<0,001) et avec la lipoprotéine Lp(a) (r=0,5 p<0,02). Le ratio Phospholipides Oxydés/apolipoprotéine B-100 est corrélé positivement avec la lipoprotéine Lp(a) (r=0,5 p<0,02). Avec le régime 2, les Phospholipides Oxydés sont corrélés positivement avec le ratio Phospholipides Oxydés/apolipoprotéine B-100 (r=0,89 p<0,0001) mais pas avec la lipoprotéine Lp(a).

### II. Mise en évidence de l'effet de l'acide punicique sur l'initiation de l'inflammation

Les inventeurs ont montré, *in vitro,* que l'acide punicique (de formule C18 : 3, n-5cis, n-7trans, n-9cis) à des doses comprises entre 5 micromolaires et 40 micromolaires permet de bloquer l'initiation de l'inflammation au niveau des polynucléaires humains. Une fois l'initiation bloquée, le taux de myéloperoxydase et le taux de phospholipides oxydés diminuent, puis les signes biologiques de l'inflammation disparaissent.

Les inventeurs ont ainsi montré l'effet, notamment, de l'acide punicique sur la résolution du Syndrome Inflammatoire chronique de bas grade.

### III. Composition alimentaire

Dans cet exemple, une composition alimentaire dite crème dessert lactovégétale est préparée à base de protéines laitières et/ou végétales, et de la composition selon l'invention. Cette composition est notamment destinée à la prévention et/ou au traitement de pathologies et/ou de troubles pouvant être améliorés ou évités par le traitement d'un syndrome inflammatoire chronique systémique de bas grade.

Il est possible d'utiliser, seules ou en mélange toute protéine d'origine alimentaire en fonction de sa double aptitude fonctionnelle (fonction organoleptique et texturante) et nutritionnelle (équilibre en acides aminés ou apport spécifique). Cet exemple peut donc s'appliquer à une composition protéique 100 % laitière, ou en mélange avec des protéines végétales (soja, pois, riz), ou en mélange avec des ovoprotéines. Ces protéines peuvent être introduites sous forme entière, partiellement hydrolysées, ou fractionnées (caséines, protéines sériques, ovoprotéines, enrichies ou pas en l'une de ses composantes).

Cette composition alimentaire se présente sous forme d'un dessert lactovégétal fermenté ou non, à texture ferme à gélifier selon la nature des additifs utilisés. Le produit est destiné à la complémentation orale. Le produit peut être supplémenté en substances nutritionnelles (vitamines, minéraux et toutes substances naturelles ou non, ayant des effets biologiques synergiques à l'activité de la composition selon l'invention telles que des acides aminés, fibres, poly phénols, caroténoïdes, stérols)

À titre d'exemple, à 49,3 kg de fromage frais à 0% de matières grasses, on incorpore 22.31 kg de matières grasses selon la composition lipidique selon l'invention, l'eau, les émulsifiants, les épaississants et le sucre, selon les règles de l'art.

Le mélange est porté à 75-80 °C sous agitation forte, en limitant l'incorporation d'air. Les arômes, et le correcteur de pH sont alors introduits. Le pH doit être compris entre 5,1 et 5,3.

Le produit est ensuite homogénéisé et stérilisé en continu et conditionné de façon aseptique ou stérilisé en discontinu après conditionnement à chaud en doses de 125 g.

| | |
|---|---|
| Composition : | |
| Fromage frais à 0% de matières grasses | 49,3 g |
| Eau | 23,70 g |
| Huile de colza | 6,30 g |
| Huile de beurre fractionnée, | |
| dite fraction oléine | 14,95 g |
| Huile de grenade | 1,06 g |
| Epaississants | 4,10 g |
| Emulsifiant | 0,10 g |
| Arômes | 0,30 g |
| Correcteurs d'acidité | 0,10 g |
| Antioxydants (tocophérols, | |
| palmitate d'ascorbyle, ac. ascorbique) | 0,03 g |

| La dite composition présente l'analyse nutritionnelle moyenne suivante : | | | | | | |
|---|---|---|---|---|---|---|
| | | | | Pour 2 desserts de 125 | | |
| | Pour 100 g de dessert | | | g : 250 g | | |
| | | | (% | | | (% |
| Valeur énergétique | 239 | kcal | AJR) | 599 | kcal | AJR) |
| | 1001 | kJ | | 2502 | kJ | |
| Protéines (100% laitière) | 4,5 | g | | 11 | g | |
| Lipides | 22,2 | g | | 56 | g | |
| dont Acides gras saturés | 10,9 | g | | 27 | g | |
| dont myristique | 1,49 | g | | 3,7 | g | |
| Acides gras monoinsaturés | 8,6 | g | | 21,4 | g | |
| Acides gras polyinsaturés | 2,8 | g | | 6,9 | g | |
| dont acide alpha-linolénique | 0,75 | g | | 1,9 | g | |
| dont acide Punicique | 0,74 | g | | 1,9 | g | |
| Glucides | 5,3 | g | | 13 | g | |
| dont sucres | 1,9 | g | | 4,9 | g | |
| Fibres | 0,13 | g | | 0,3 | g | |
| Calcium | 88 | mg | 11 | 220 | mg | 27 |
| Phosphore | 65 | mg | | 161 | mg | 29 |
| Magnésium | 7 | mg | | 18 | mg | 12 |
| Sodium | 41 | mg | | 102 | mg | |
| Potassium | 56 | mg | | 141 | mg | |

### IV. Complément alimentaire

Cet exemple vise une composition nutritionnelle adaptée à la complémentation orale protéique, notamment des personnes âgées dénutries et comprenant la composition selon l'invention.

Ce complément alimentaire peut être consommé lors de collations ou d'un repas. En particulier, cette composition nutritionnelle peut être administrée 3 fois par jour à une dose de 70 grammes, soit 60 g de protéines par jour, apportant ainsi 977 Kcalories par jour (pour 210 grammes de complément sous forme de poudre), dont environ 28 Kcalories provenant de l'acide myristique, environ 14 Kcalories provenant de l'acide alpha-linolénique, environ 15 Kcalories provenant de l'acide punicique. Les besoins protéiques des personnes âgées peuvent atteindre 1,5 g/kg/jour soit 110 à 120g par jour pour une personne de 80 kg.

Ce complément alimentaire peut être ajouté à différents aliments tels que des crèmes, boisson laitière, yaourts, potages, sauces.

Ajoutée à de l'eau (250 ml d'eau et 70 g de poudre), la préparation apporte 7 g de protéines par 100 ml de boisson.

Ce complément alimentaire peut être disponible sous différentes aromatisations : vanille, chocolat, fraise, citron, fruits exotiques. Il peut se présenter sous différentes textures, épaisse, gélifiée, liquide par ajout de texturants de type carraghénanes, alginates, gomme xanthane, amidon modifié.

**Composition centésimale :**

| | |
|---|---|
| Protéines de lait | 53,26 % |
| Protéines de soja | 11,64% |
| Protéines de blanc d'oeuf | 11,51 % |
| Protéines de riz | 7,75% |
| Composition lipidique selon l'invention | 9,5% |
| Edulcorants | 1,18% |
| Mélange de vitamines (12) | 0,26% |
| Mélange de minéraux (11) | 0,68% |
| Saccharose | 3,1% |
| Poudre de vanille | 0,19% |
| Colorant naturel | 0,065% |
| Arômes | 1,9% |

Le complément alimentaire peut être obtenu selon diverses techniques connues de l'Homme du Métier.

Par exemple, on peut d'abord procéder au mélange de protéines et de saccharose par voie sèche. Ce mélange peut être soumis à une granulation par voie sèche. Par exemple on peut utiliser des granulateurs de type Glatt, procédé discontinu. Il peut être soumis à une granulation après avoir été préalablement réhydraté.

La composition selon l'invention, est pulvérisée sur le mélange précédent, lors de l'opération de granulation ou postérieurement. Les autres ingrédients sont ensuite ajoutés au mélange protides-lipides-glucides pour constituer le produit final. La composition selon l'invention étant riche en acides gras insaturés en n-3, l'ensemble du procédé est réalisé à température modérée inférieure à 70°C. Les temps de fabrication sont courts, inférieurs à quelques minutes.

Le produit est ensuite conditionné dans des emballages particulièrement étanches à l'air, sous atmosphère contrôlée.

70 g de complément nutritionnel oral sera reconstitué dans un volume d'eau de 250 ml faiblement minéralisée. L'osmolarité du produit ainsi reconstitué sera hypo osmolaire comprise entre 150 et 200 mosmoles/litre.

**Composition nutritionnelle du complément alimentaire pour 100 g:**

| **ANALYSE NUTRITIONNELLE MOYENNE** | | | | | |
|---|---|---|---|---|---|
| | Pour 100 g de poudre de complément alimentaire | | | Pour 3 prises de 250 ml (210 g de poudre) | |
| | | | %AET (% de l'Apport énergétique total apportée par la poudre) | | |
| | 465 | kcal | | 977 | kcal |
| Valeur énergétique | 1944 | kJ | | 4083 | kJ |
| Protéines | 32,7 | g | 28 | 69 | g |
| Lipides | 19,9 | g | 38 | 41,7 | g |
| dont Acides gras | | | | | |
| saturés | 8,5 | g | | 17,9 | g |
| dont l'acide | | | | | |
| myristique | 1,49 | g | 2,9 | 3,1 | g |
| Acides gras | | | | | |
| monoinsaturés | 7,51 | g | | 15,8 | g |
| Acides gras | | | | | |
| polyinsaturés | 3,81 | g | | 8,0 | g |
| dont l'acide alpha | | | | | |
| linolénique | 0,80 | g | 1,5 | 1,67 | g |
| dont l'acide | | | | | |
| Punicique | 0,84 | g | 1,6 | 1,76 | g |
| Glucides | 38,8 | g | 33 | 81,5 | g |
| dont sucres | 18,1 | g | | 37,9 | g |
| Fibres | 0,14 | g | | 0,3 | g |
| | | | | | |
| Calcium | 419 | mg | | 879 | mg |
| Phosphore | 358 | mg | | 751 | mg |
| Magnésium | 95 | mg | | 199 | mg |
| Sodium | 165 | mg | | 347 | mg |
| Potassium | 789 | mg | | 1658 | mg |
| Fer | 7,46 | mg | | 15,7 | mg |
| Zinc | 7,46 | mg | | 15,7 | mg |
| Cuivre | 0,53 | mg | | 1,1 | mg |
| Manganèse | 0,60 | mg | | 1,3 | mg |
| Iode | 78 | µg | | 164,3 | µg |
| Sélénium | 30 | µg | | 62,5 | µg |
| Vit A | 235 | µg | | 494 | µg |
| Vit D | 1,3 | µg | | 2,7 | µg |
| Vit E | 4,1 | mg | | 8,5 | mg |
| Vit C | 31 | mg | | 64 | mg |
| Vit B1 | 0,6 | mg | | 1,3 | mg |
| Vit B2 | 0,6 | mg | | 1,2 | mg |
| Vit B6 | 1,0 | mg | | 2,1 | mg |
| Vit PP | 6 | mg | | 12,9 | mg |
| Vit B5 | 1,5 | mg | | 3,2 | mg |
| Vit B12 | 2,3 | mg | | 4,8 | mg |
| Vit B9 | 138 | µg | | 289 | µg |
| Vit B8 | 5 | µg | | 10,8 | µg |

## Revendications

1. Composition comprenant :
- de l'acide myristique ; et
- entre 10 et 70 %, de préférence entre 30 et 65 % et tout préférentiellement entre 50 et 60 % en poids par rapport au poids de l'acide myristique, d'un acide gras X1 et/ou d'au moins un de ses sels, ledit acide gras X1 comprenant de 16 à 18 atomes de carbone et un diène conjugué n-5cis, n-7trans ; et/ou
- entre 10 et 70 % de préférence entre 30 et 65 % et tout préférentiellement entre 50 et 60 % en poids par rapport au poids de l'acide myristique, d'un acide gras X2 et/ou d'au moins un de ses sels, ledit acide gras X2 comprenant de 18 à 22 atomes de carbone et un triène conjugué n-5cis, n-7trans, n-9cis.

2. Composition selon la revendication 1, dans laquelle au moins une partie de l'acide myristique compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 %, et tout préférentiellement au moins 70 % de l'acide myristique estérifié à des chaînes de glycérol est en position sn-2.

3. Composition selon l'une des revendications 1 ou 2, dans laquelle au moins une partie de l'acide gras X1 compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 %, et tout préférentiellement au moins 70 % de l'acide gras X1 estérifié à des chaînes de glycérol est en position sn-2.

4. Composition selon l'une quelconque des revendications 1 à 3, dans laquelle au moins une partie de l'acide gras X2 compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 %, et tout préférentiellement au moins 70 % de l'acide gras X2 estérifié à des chaînes de glycérol est en position sn-2.

5. Composition selon l'une quelconque des revendications 1 à 4, **caractérisée en ce que** les sels d'acides gras X1 et X2 sont choisis dans le groupe comprenant les sels de métaux alcalins, les sels de métaux alcalino-terreux et les sels d'acides aminés, de préférence les sels d'acides aminés et tout préférentiellement les sels d'arginine et de lysine.

6. Composition selon l'une quelconque des revendications 1 à 5, **caractérisée en ce que** la quantité d'acide myristique dans ladite composition correspond à au moins 1,2 %, de préférence de 1,2 à 1,8 % et tout préférentiellement à 1,5 % de l'apport énergétique total journalier recommandé pour un être humain.

7. Composition selon la revendication 6, **caractérisée en ce que** la quantité d'acide myristique dans ladite composition est comprise 1,6 et 8,8 grammes, de préférence entre 1,9 et 6,4 grammes et tout préférentiellement entre 2,9 et 4,3 grammes.

8. Composition selon l'une quelconque des revendications 1 à 7, **caractérisée en ce que** la quantité d'acide gras X1 et/ou d'au moins un de ses sels dans ladite composition correspond à au moins 0,15 %, de préférence à 0,3 à 0,9 % et tout préférentiellement à 0,4 à 0,8 % de l'apport énergétique total journalier recommandé pour un être humain.

9. Composition selon la revendication 8, **caractérisée en ce que** la quantité d'acide gras X1 et/ou d'au moins un de ses sels dans ladite composition est comprise entre 0,4 et 4 grammes, de préférence entre 0,6 et 3 grammes et tout préférentiellement entre 1 et 2 grammes.

10. Composition selon l'une quelconque des revendications 1 à 9, **caractérisée en ce que** la quantité d'acide gras X2 et/ou d'au moins un de ses sels dans ladite composition correspond à au moins 0,3 %, de préférence à 0,3 à 0,9 % et tout préférentiellement à 0,4 à 0,8 % de l'apport énergétique total journalier recommandé pour un être humain.

11. Composition selon la revendication 10, **caractérisée en ce que** la quantité d'acide gras X2 et/ou d'au moins un de ses sels dans ladite composition est comprise 0,5 et 4 grammes, de préférence entre 0,6 et 3 grammes et tout préférentiellement entre 1 et 2 grammes.

12. Composition selon l'une quelconque des revendications 1 à 11, **caractérisée en ce que** ledit acide gras X2, est l'acide punicique.

13. Composition selon la revendication 12, **caractérisée en ce qu'**au moins une partie de l'acide punicique compris dans la composition est issue d'au moins un végétal choisi dans le groupe comprenant les végétaux de la famille des Cucurbitaceae, notamment *Trichosanthes cucumerina anguina, Momordica charantia, Cucurbita maxima, Citrullus lanatus* et les végétaux de la famille des Punicaceae, notamment *Punica granatum.*

14. Composition selon l'une quelconque des revendications 1 à 13, **caractérisée en ce qu'**elle comprend en outre :
- entre 45 et 75 % de préférence entre 50 et 70 % et tout préférentiellement entre 55 et 68 % en poids par rapport au poids de l'acide myristique, d'acide alpha-linolénique.

15. Composition selon la revendication 14, **caractérisée en ce qu'**au moins une partie de l'acide alpha-linolénique compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 % et tout préférentiellement au moins 70 % de l'acide alpha-linolénique estérifié à des chaînes de glycérol est en position sn-2.

16. Composition selon l'une des revendications 14 ou 15, **caractérisée en ce que** la quantité d'acide alpha-linolénique dans ladite composition correspond à au moins 0,7 %, de préférence à 0,7 à 1,1 % et tout préférentiellement à 0,9 % de l'apport énergétique total journalier recommandé pour un être humain.

17. Composition selon la revendication 16, **caractérisée en ce que** la quantité d'acide alpha-linolénique dans ladite composition est comprise entre 1 et 4 grammes, de préférence entre 1,5 et 3 grammes et tout préférentiellement de 2 grammes.

18. Composition selon l'une quelconque des revendications 14 à 17, **caractérisée en ce qu'**au moins une partie de l'acide alpha-linolénique compris dans la composition est issue d'au moins un végétal choisi dans le groupe comprenant *Brassica napus* et les végétaux du genre *Juglans,* de préférence *Brassica napus.*

19. Composition selon l'une quelconque des revendications 1 à 18, **caractérisée en ce qu'**au moins une partie de l'acide myristique compris dans la composition, et/ou au moins une partie de l'acide gras X1 compris dans la composition, sont issues d'au moins une matière grasse d'origine animale, notamment de matière grasse lactique, et/ou d'origine végétale.

20. Composition selon l'une quelconque des revendications 1 à 19, **caractérisée en ce qu'**elle comprend en outre :
- entre 15 et 70 %, de préférence entre 25 et 40 % et tout préférentiellement entre 30 et 35 % en poids par rapport au poids de l'acide myristique, d'acide stéaridonique.

21. Composition selon la revendication 20, **caractérisée en ce qu'**au moins une partie de l'acide stéaridonique compris dans la composition est estérifiée à des chaînes de glycérol, dans une configuration stéréochimique choisie parmi : sn-1,2,3 ; sn-1,2 ; sn-1,3 ; sn-2,3 ; sn-1 ; sn-2 et sn-3 afin de former des triacylglycérols, dans lesquels au moins 50 %, de préférence au moins 60 %, et tout préférentiellement au moins 70 % de l'acide stéaridonique estérifié à des chaînes de glycérol est en position sn-2.

22. Composition selon l'une des revendications 20 ou 21, **caractérisée en ce que** la quantité d'acide stéaridonique dans ladite composition correspond à au moins 0,25 %, de préférence à 0,25 à 0,9 % et tout préférentiellement à 0,3 à 0,8 % de l'apport énergétique total journalier recommandé pour un être humain.

23. Composition selon la revendication 22, **caractérisée en ce que** la quantité d'acide stéaridonique dans ladite composition est comprise entre 0,4 et 4 grammes de préférence entre 0,55 et 3,2 grammes et tout préférentiellement entre 0,8 et 2 grammes.

24. Composition selon l'un quelconque des revendications 6, 8, 10, 16 ou 22, **caractérisée en ce que** l'apport énergétique total journalier recommandé pour un être humain est compris entre 1800 et 2500 Kcalories, de préférence entre 2000 et 2400 Kcalories et tout préférentiellement 2200 Kcalories dont entre 25 et 40 %, de préférence entre 30 et 37 % et tout préférentiellement 33 % de l'énergie provient de lipides.

25. Composition selon l'une quelconque des revendications 6 à 11, 16, 17, 22 ou 23 **caractérisée en ce qu'**elle constitue une dose journalière.

26. Composition selon l'une quelconque des revendications 1 à 25, **caractérisée en ce que** la composition est choisie dans le groupe comprenant les compositions alimentaires, notamment diététique, les compositions pharmaceutiques et les compositions nutraceutiques.

27. Composition selon la revendication 26, pour son utilisation dans la préparation d'une composition alimentaire, pharmaceutique ou nutraceutique destinée à la prévention et/ou au traitement de syndrome inflammatoire chronique systémique de bas grade.

28. Composition pour son utilisation selon la revendication 27, **caractérisée en ce que** la composition est destinée à être administrée à des sujets présentant une surcharge pondérale et/ou à des sujets âgés et/ou à des sujets atteints d'une pathologie choisie dans le groupe comprenant l'obésité, le syndrome métabolique, l'insulinorésistance, les diabètes, l'athérosclérose, les maladies cardiovasculaires, la dégénérescence cérébrale, la maladie d'Alzheimer, les accidents vasculaires cérébraux.

29. Composition pour son utilisation selon la revendication 28 **caractérisée en ce que** les pathologies sont choisies dans le groupe comprenant l'obésité, le syndrome métabolique, l'insulinorésistance, les diabètes, l'athérosclérose, , la dégénérescence cérébrale, la maladie d'Alzheimer, les accidents vasculaires cérébraux.

## Patentansprüche

1. Zusammensetzung, umfassend:
- Myristinsäure; und
- eine Fettsäure X1 und/oder mindestens eines ihrer Salze in einer Menge von zwischen 10 und 70 Gew.-%, vorzugsweise zwischen 30 und 65 Gew.-% und besonders bevorzugt zwischen 50 und 60 Gew.-% in Bezug auf das Gewicht der Myristinsäure, wobei die Fettsäure X1 16 bis 18 Kohlenstoffatome und ein n-5cis-, n-7trans-konjugiertes Dien umfasst; und/oder
- eine Fettsäure X2 und/oder mindestens eines ihrer Salze in einer Menge von zwischen 10 und 70 Gew.-%, vorzugsweise zwischen 30 und 65 Gew.-% und besonders bevorzugt zwischen 50 und 60 Gew.-% in Bezug auf das Gewicht der Myristinsäure, wobei die Fettsäure X2 18 bis 22 Kohlenstoffatome und ein n-5cis-, n-7trans-, n-9cis-konjugiertes Trien umfasst.

2. Zusammensetzung nach Anspruch 1, in der mindestens ein Teil der in der Zusammensetzung vorhandenen Myristinsäure mit Glycerinketten in einer stereochemischen Konfiguration, ausgewählt aus: sn-1,2,3; sn-1,2; sn-1,3; sn-2,3; sn-1; sn-2 und sn-3, unter Bildung von Triacylglycerinen verestert ist, in denen sich mindestens 50%, vorzugsweise mindestens 60% und besonders bevorzugt mindestens 70% der mit Glycerinketten veresterten Myristinsäure in sn-2-Position befindet.

3. Zusammensetzung nach einem der Ansprüche 1 oder 2, in der mindestens ein Teil der in der Zusammensetzung vorhandenen Fettsäure X1 mit Glycerinketten in einer stereochemischen Konfiguration, ausgewählt aus: sn-1,2,3; sn-1,2; sn-1,3; sn-2,3; sn-1; sn-2 und sn-3, unter Bildung von Triacylglycerinen verestert ist, in denen sich mindestens 50%, vorzugsweise mindestens 60% und besonders bevorzugt mindestens 70% der mit Glycerinketten veresterten Fettsäure X1 in sn-2-Position befindet.

4. Zusammensetzung nach einem der Ansprüche 1 bis 3, in der mindestens ein Teil der in der Zusammensetzung vorhandenen Fettsäure X2 mit Glycerinketten in einer stereochemischen Konfiguration, ausgewählt aus: sn-1,2,3; sn-1,2; sn-1,3; sn-2,3; sn-1; sn-2 und sn-3, unter Bildung von Triacylglycerinen verestert ist, in denen sich mindestens 50%, vorzugsweise mindestens 60% und besonders bevorzugt mindestens 70% der mit Glycerinketten veresterten Fettsäure X2 in sn-2-Position befindet.

5. Zusammensetzung nach einem der Ansprüche 1 bis 4, **dadurch gekennzeichnet, dass** die Salze der Fettsäuren X1 und X2 aus der Gruppe umfassend die Alkalimetallsalze, Erdalkalimetallsalze und Salze von Aminosäuren, vorzugsweise die Salze von Aminosäuren und besonders bevorzugt die Salze von Arginin und Lysin, ausgewählt sind.

6. Zusammensetzung nach einem der Ansprüche 1 bis 5, **dadurch gekennzeichnet, dass** die Menge an Myristinsäure in der Zusammensetzung mindestens 1,2%, vorzugsweise 1,2 bis 1,8% und besonders bevorzugt 1,5% der für einen Menschen empfohlenen Gesamtenergiezufuhr pro Tag entspricht.

7. Zusammensetzung nach Anspruch 6, **dadurch gekennzeichnet, dass** die Menge an Myristinsäure in der Zusammensetzung zwischen 1,6 und 8,8 Gramm, vorzugsweise zwischen 1,9 und 6,4 Gramm und besonders bevorzugt zwischen 2,9 und 4,3 Gramm beträgt.

8. Zusammensetzung nach einem der Ansprüche 1 bis 7, **dadurch gekennzeichnet, dass** die Menge an Fettsäure X1 und/oder an mindestens einem ihrer Salze in der Zusammensetzung mindestens 0,15%, vorzugsweise 0,3 bis 0,9% und besonders bevorzugt 0,4 bis 0,8% der für einen Menschen empfohlenen Gesamtenergiezufuhr pro Tag entspricht.

9. Zusammensetzung nach Anspruch 8, **dadurch gekennzeichnet, dass** die Menge an Fettsäure X1 und/oder an mindestens einem ihrer Salze in der Zusammensetzung zwischen 0,4 und 4 Gramm, vorzugsweise zwischen 0,6 und 3 Gramm und besonders bevorzugt zwischen 1 und 2 Gramm beträgt.

10. Zusammensetzung nach einem der Ansprüche 1 bis 9, **dadurch gekennzeichnet, dass** die Menge an Fettsäure X2 und/oder an mindestens einem ihrer Salze in der Zusammensetzung mindestens 0,3%, vorzugsweise 0,3 bis 0,9% und besonders bevorzugt 0,4 bis 0,8% der für einen Menschen empfohlenen Gesamtenergiezufuhr pro Tag entspricht.

11. Zusammensetzung nach Anspruch 10, **dadurch gekennzeichnet, dass** die Menge an Fettsäure X2 und/oder an mindestens einem ihrer Salze in der Zusammensetzung zwischen 0,5 und 4 Gramm, vorzugsweise zwischen 0,6 und 3 Gramm und besonders bevorzugt zwischen 1 und 2 Gramm beträgt.

12. Zusammensetzung nach einem der Ansprüche 1 bis 11, **dadurch gekennzeichnet, dass** es sich bei der Fettsäure X2 um die Punicinsäure handelt.

13. Zusammensetzung nach Anspruch 12, **dadurch gekennzeichnet, dass** mindestens ein Teil der in der Zusammensetzung vorhandenen Punicinsäure aus mindestens einer Pflanze, ausgewählt aus der Gruppe umfassend die Pflanzen der Familie Cucurbitaceae, insbesondere *Trichosanthes cucumerina anguina, Momordica charantia, Cucurbita maxima, Citrullus lanatus,* und die Pflanzen der Familie Punicaceae, insbesondere *Punica granatum,* stammt.

14. Zusammensetzung nach einem der Ansprüche 1 bis 13, **dadurch gekennzeichnet, dass** sie weiterhin Folgendes umfasst:
- alpha-Linolensäure in einer Menge von zwischen 45 und 75 Gew.-%, vorzugsweise zwischen 50 und 70 Gew.-% und besonders bevorzugt zwischen 55 und 68 Gew.-% in Bezug auf das Gewicht der Myristinsäure.

15. Zusammensetzung nach Anspruch 14, **dadurch gekennzeichnet, dass** mindestens ein Teil der in der Zusammensetzung vorhandenen alpha-Linolensäure mit Glycerinketten in einer stereochemischen Konfiguration, ausgewählt aus: sn-1,2,3; sn-1,2; sn-1,3; sn-2,3; sn-1; sn-2 und sn-3, unter Bildung von Triacylglycerinen verestert ist, in denen sich mindestens 50%, vorzugsweise mindestens 60% und besonders bevorzugt mindestens 70% der mit Glycerinketten veresterten alpha-Linonlensäure in sn-2-Position befindet.

16. Zusammensetzung nach einem der Ansprüche 14 oder 15, **dadurch gekennzeichnet, dass** die Menge der alpha-Linolensäure in der Zusammensetzung mindestens 0,7%, vorzugsweise 0,7 bis 1,1% und besonders bevorzugt 0,9% der für einen Menschen empfohlenen Gesamtenergiezufuhr pro Tag entspricht.

17. Zusammensetzung nach Anspruch 16, **dadurch gekennzeichnet, dass** die Menge der alpha-Linolensäure in der Zusammensetzung zwischen 1 und 4 Gramm, vorzugsweise zwischen 1,5 und 3 Gramm und besonders bevorzugt 2 Gramm beträgt.

18. Zusammensetzung nach einem der Ansprüche 14 bis 17, **dadurch gekennzeichnet, dass** mindestens ein Teil der in der Zusammensetzung vorhandenen alpha-Linolensäure aus mindestens einer Pflanze, ausgewählt aus der Gruppe umfassend *Brassica napus* und die Pflanzen der Gattung *Juglans,* vorzugsweise *Brassica napus,* stammt.

19. Zusammensetzung nach einem der Ansprüche 1 bis 18, **dadurch gekennzeichnet, dass** mindestens ein Teil der in der Zusammensetzung vorhandenen Myristinsäure und/oder mindestens ein Teil der in der Zusammensetzung vorhandenen Fettsäure X1 aus mindestens einem Fett tierischen Ursprungs, insbesondere aus Milchfett und/oder Fett pflanzlichen Ursprungs, stammen.

20. Zusammensetzung nach einem der Ansprüche 1 bis 19, **dadurch gekennzeichnet, dass** sie weiterhin Folgendes umfasst:
- Stearidonsäure in einer Menge von zwischen 15 und 70 Gew.-%, vorzugsweise zwischen 25 und 40 Gew.-% und besonders bevorzugt zwischen 30 und 35 Gew.-% in Bezug auf das Gewicht der Myristinsäure.

21. Zusammensetzung nach Anspruch 20, **dadurch gekennzeichnet, dass** mindestens ein Teil der in der Zusammensetzung vorhandenen Stearidonsäure mit Glycerinketten in einer stereochemischen Konfiguration, ausgewählt aus: sn-1,2,3; sn-1,2; sn-1,3; sn-2,3; sn-1; sn-2 und sn-3, unter Bildung von Triacylglycerinen verestert ist, in denen sich mindestens 50%, vorzugsweise mindestens 60% und besonders bevorzugt mindestens 70% der mit Glycerinketten veresterten Stearidonsäure in sn-2-Position befindet.

22. Zusammensetzung nach einem der Ansprüche 20 oder 21, **dadurch gekennzeichnet, dass** die Menge an Stearidonsäure in der Zusammensetzung mindestens 0,25%, vorzugsweise 0,25 bis 0,9% und besonders bevorzugt 0,3 bis 0,8% der für einen Menschen empfohlenen Gesamtenergiezufuhr pro Tag entspricht.

23. Zusammensetzung nach Anspruch 22, **dadurch gekennzeichnet, dass** die Menge an Stearidonsäure in der Zusammensetzung zwischen 0,4 und 4 Gramm, vorzugsweise zwischen 0,55 und 3,2 Gramm und besonders bevorzugt zwischen 0,8 und 2 Gramm beträgt.

24. Zusammensetzung nach einem der Ansprüche 6, 8, 10, 16 oder 22, **dadurch gekennzeichnet, dass** die für einen Menschen empfohlene Gesamtenergiezufuhr pro Tag zwischen 1800 und 2500 Kilokalorien, vorzugsweise zwischen 2000 und 2400 Kilokalorien und besonders bevorzugt 2200 Kilokalorien beträgt, wobei zwischen 25 und 40%, vorzugsweise zwischen 30 und 37% und besonders bevorzugt 33% der Energie aus Lipiden stammt.

25. Zusammensetzung nach einem der Ansprüche 6 bis 11, 16, 17, 22 oder 23, **dadurch gekennzeichnet, dass** sie eine Tagesdosis darstellt.

26. Zusammensetzung nach einem der Ansprüche 1 bis 25, **dadurch gekennzeichnet, dass** die Zusammensetzung aus der Gruppe umfassend Nährmittelzusammensetzungen, insbesondere diätetische Nährmittelzusammensetzungen, pharmazeutische Zusammensetzungen und nutrazeutische Zusammensetzungen ausgewählt ist.

27. Zusammensetzung nach Anspruch 26 zur Verwendung in der Herstellung einer Nährmittelzusammensetzung, pharmazeutischen Zusammensetzung oder nutrazeutischen Zusammensetzung zur Vorbeugung und/oder Behandlung von niedriggradigem chronischsystemischem Entzündungssyndrom.

28. Zusammensetzung zur Verwendung nach Anspruch 27, **dadurch gekennzeichnet, dass** die Zusammensetzung an übergewichtige Individuen und/oder ältere Individuen und/oder Individuen, die an einer Krankheit aus der Gruppe umfassend Adipositas, metabolisches Syndrom, Insulinresistenz, Diabetes, Atherosklerose, Herz-Kreislauf-Krankheiten, zerebrale Degeneration, Morbus Alzheimer und Schlaganfälle leiden, zu verabreichen ist.

29. Zusammensetzung zur Verwendung nach Anspruch 28, **dadurch gekennzeichnet, dass** die Krankheiten aus der Gruppe umfassend Adipositas, metabolisches Syndrom, Insulinresistenz, Diabetes, Atherosklerose, zerebrale Degeneration, Morbus Alzheimer und Schlaganfälle ausgewählt sind.

## Claims

1. Composition comprising:
- myristic acid; and
- between 10 and 70%, preferably between 30 and 65%, and most preferably between 50 and 60% by weight in relation to the weight of the myristic acid, of a fatty acid X1 and/or at least one of the salts thereof, said fatty acid X1 comprising from 16 to 18 carbon atoms and an n-5cis, n-7trans conjugated diene; and/or
- between 10 and 70%, preferably between 30 and 65%, and most preferably between 50 and 60% by weight in relation to the weight of the myristic acid, of a fatty acid X2 and/or at least one of the salts thereof, said fatty acid X2 comprising from 18 to 22 carbon atoms and an n-5cis, n-7trans, n-9cis conjugated triene.

2. Composition according to Claim 1, wherein at least part of the myristic acid contained in the composition is esterified at glycerol chains, in a stereochemical configuration which is selected from: sn-1,2,3; sn-1,2; sn-1,3; sn-2,3; sn-1; sn-2 and sn-3 in order to form triacylglycerols, wherein at least 50%, preferably at least 60%, and most preferably at least 70% of the myristic acid esterified at glycerol chains is in the sn-2 position.

3. Composition according to either Claim 1 or Claim 2, wherein at least part of the fatty acid X1 contained in the composition is esterified at glycerol chains, in a stereochemical configuration which is selected from: sn-1,2,3; sn-1,2; sn-1,3; sn-2,3; sn-1; sn-2 and sn-3 in order to form triacylglycerols, wherein at least 50%, preferably at least 60%, and most preferably at least 70% of the fatty acid X1 esterified at glycerol chains is in the sn-2 position.

4. Composition according to any one of Claims 1 to 3, wherein at least part of the fatty acid X2 contained in the composition is esterified at glycerol chains, in a stereochemical configuration which is selected from: sn-1,2,3; sn-1,2; sn-1,3; sn-2,3; sn-1; sn-2 and sn-3 in order to form triacylglycerols, wherein at least 50%, preferably at least 60%, and most preferably at least 70% of the fatty acid X2 esterified at glycerol chains is in the sn-2 position.

5. Composition according to any one of Claims 1 to 4, **characterised in that** the salts of fatty acids X1 and X2 are selected from the group comprising alkali metal salts, alkaline earth metal salts and amino acid salts, preferably amino acid salts, and most preferably arginine and lysine salts.

6. Composition according to any one of Claims 1 to 5, **characterised in that** the amount of myristic acid in said composition corresponds to at least 1.2%, preferably to from 1.2 to 1.8%, and most preferably to 1.5% of the recommended total daily energy intake for a human being.

7. Composition according to Claim 6, **characterised in that** the amount of myristic acid in said composition is between 1.6 and 8.8 grams, preferably between 1.9 and 6.4 grams, and most preferably between 2.9 and 4.3 grams.

8. Composition according to any one of Claims 1 to 7, **characterised in that** the amount of fatty acid X1 and/or at least one of the salts thereof in said composition corresponds to at least 0.15%, preferably to from 0.3 to 0.9%, and most preferably to from 0.4 to 0.8% of the recommended total daily energy intake for a human being.

9. Composition according to Claim 8, **characterised in that** the amount of fatty acid X1 and/or at least one of the salts thereof in said composition is between 0.4 and 4 grams, preferably between 0.6 and 3 grams, and most preferably between 1 and 2 grams.

10. Composition according to any one of Claims 1 to 9, **characterised in that** the amount of fatty acid X2 and/or at least one of the salts thereof in said composition corresponds to at least 0.3%, preferably to from 0.3 to 0.9%, and most preferably to from 0.4 to 0.8% of the recommended total daily energy intake for a human being.

11. Composition according to Claim 10, **characterised in that** the amount of fatty acid X2 and/or at least one of the salts thereof in said composition is between 0.5 and 4 grams, preferably between 0.6 and 3 grams, and most preferably between 1 and 2 grams.

12. Composition according to any one of Claims 1 to 11, **characterised in that** said fatty acid X2 is punicic acid.

13. Composition according to Claim 12, **characterised in that** at least part of the punicic acid contained in the composition is derived from at least one plant selected from the group comprising the plants from the family of Cucurbitaceae, in particular *Trichosanthes cucumerina anguina, Momordica charantia, Cucurbita maxima, Citrullus lanatus* and the plants from the family of Punicaceae, in particular *Punica granatum.*

14. Composition according to any one of Claims 1 to 13, **characterised in that** it further comprises:
- between 45 and 75%, preferably between 50 and 70%, and most preferably between 55 and 68% by weight, in relation to the weight of the myristic acid, of alpha-linolenic acid.

15. Composition according to Claim 14, **characterised in that** at least part of the alpha-linolenic acid contained in the composition is esterified at glycerol chains, in a stereochemical configuration which is selected from: sn-1,2,3; sn-1,2; sn-1,3; sn-2,3; sn-1; sn-2 and sn-3 in order to form triacylglycerols, wherein at least 50%, preferably at least 60%, and most preferably at least 70% of the alpha-linolenic acid esterified at glycerol chains is in the sn-2 position.

16. Composition according to either Claim 14 or Claim 15, **characterised in that** the amount of alpha-linolenic acid in said composition corresponds to at least 0.7%, preferably to from 0.7 to 1.1%, and most preferably to 0.9% of the recommended total daily energy intake for a human being.

17. Composition according to Claim 16, **characterised in that** the amount of alpha-linolenic acid in said composition is between 1 and 4 grams, preferably between 1.5 and 3 grams, and most preferably 2 grams.

18. Composition according to any one of Claims 14 to 17, **characterised in that** at least part of the alpha-linolenic acid contained in the composition is derived from at least one plant which is selected from the group comprising *Brassica napus* and the plants of the genus *Juglans,* preferably *Brassica napus.*

19. Composition according to any one of Claims 1 to 18, **characterised in that** at least part of the myristic acid contained in the composition and/or at least part of the fatty acid X1 contained in the composition are derived from at least one fat of animal origin, in particular milk fat, and/or of plant origin.

20. Composition according to any one of Claims 1 to 19, **characterised in that** it further comprises:
- between 15 and 70%, preferably between 25 and 40%, and most preferably between 30 and 35% by weight, in relation to the weight of the myristic acid, of stearidonic acid.

21. Composition according to Claim 20, **characterised in that** at least part of the stearidonic acid contained in the composition is esterified at glycerol chains, in a stereochemical configuration which is selected from: sn-1,2,3; sn-1,2; sn-1,3; sn-2,3; sn-1; sn-2 and sn-3 in order to form triacylglycerols, wherein at least 50%, preferably at least 60%, and most preferably at least 70% of the stearidonic acid esterified at glycerol chains is in the sn-2 position.

22. Composition according to either Claim 20 or Claim 21, **characterised in that** the amount of stearidonic acid in said composition corresponds to at least 0.25%, preferably to from 0.25 to 0.9%, and most preferably to from 0.3 to 0.8% of the recommended total daily energy intake for a human being.

23. Composition according to Claim 22, **characterised in that** the amount of stearidonic acid in said composition is between 0.4 and 4 grams, preferably between 0.55 and 3.2 grams, and most preferably between 0.8 and 2 grams.

24. Composition according to any one of Claims 6, 8, 10, 16, or 22, **characterised in that** the recommended total daily energy intake for a human being is between 1800 and 2500 kilocalories, preferably between 2000 and 2400 kilocalories, and most preferably 2200 kilocalories, of which between 25 and 40%, preferably between 30 and 37%, and most preferably 33% of the energy comes from lipids.

25. Composition according to any one of Claims 6 to 11, 16, 17, 22 or 23, **characterised in that** it constitutes a daily dose.

26. Composition according to any one of Claims 1 to 25, **characterised in that** the composition is selected from the group comprising food, in particular dietary, compositions, pharmaceutical compositions and nutraceutical compositions.

27. Composition according to Claim 26, for use in the preparation of a food, pharmaceutical or nutraceutical composition which is intended to prevent and/or treat low-grade chronic, systemic inflammatory response syndrome.

28. Composition for use according to Claim 27, **characterised in that** the composition is to be administered to overweight subjects and/or to elderly subjects and/or to subjects suffering from a pathology which is selected from the group comprising obesity, metabolic syndrome, insulin resistance, diabetes, atherosclerosis, cardiovascular diseases, cerebral degeneration, Alzheimer's disease, cerebrovascular accidents.

29. Composition for use according to Claim 28, **characterised in that** the pathologies are selected from the group comprising obesity, metabolic syndrome, insulin resistance, diabetes, atherosclerosis, cerebral degeneration, Alzheimer's disease, cerebrovascular accidents.
